# EUROPEAN PATENT APPLICATION

(11) **EP 3 777 887 A1**
(43) Date of publication of application: **17.02.2021**
(21) Application number: 19776917.7
(22) Date of filing: 28.03.2019
(51) Int. Cl.: A61K 39/39, A61K 39/35, A61P 11/06, A61P 37/04, A61P 37/08, A61P 43/00, A61K 31/728

(54) **ALLERGENIC ACTION ENHANCER COMPRISING HYALURONIC ACID AS ACTIVE INGREDIENT**

(30) Priority: 30.03.2018 JP 2018066752
(71) Applicant: Kawasaki Gakuen Educational Foundation, Kurashiki-shi, Okayama 701-0192 (JP)
(72) Inventor: KATOH Shigeki, Kurashiki-shi, Okayama 701-0192 (JP)
(74) Representative: Brantsandpatents bvba
(86) International application number: PCT/JP2019/013838
(87) International publication number: WO 2019/189676

(57) **Abstract**

It is an object to enhance the action of an allergen in sublingual immunotherapy for an asthma patient, and to reduce the amount of the allergen in order to reduce side effects of the allergen in the sublingual immunotherapy. Provided is an allergenic action enhancer to be administered in combined use with an allergen in sublingual immunotherapy (SLIT), the allergenic action enhancer containing hyaluronic acid (HA) as an active ingredient.

## Description

### TECHNICAL FIELD

The present invention relates to an allergenic action enhancer containing hyaluronic acid as an active ingredient, and to a pharmaceutical composition for oromucosal administration containing an allergen and hyaluronic acid having an allergenic action-enhancing ability as active ingredients.

### BACKGROUND ART

The number of patients with bronchial asthma (asthma) in Japan is on the increase at from 7% to 10% of its population, and more than 1,500 people die of an asthma attack every year. Mites account for more than half of allergens that cause asthma in Japan. All current standard treatments for asthma are symptomatic therapies, and a novel treatment method needs to be developed. Allergen-specific immunotherapy is expected to be definitive therapy for asthma, but still has many problems in terms of efficacy and safety. In recent years, among the allergen-specific immunotherapies, sublingual immunotherapy (SLIT) has been attracting attention.

In recent years, mite allergen-specific sublingual immunotherapy has been performed for patients with allergic rhinitis and asthma in Japan. The mite allergen-specific sublingual immunotherapy has been found to have efficacy for allergic rhinitis, and has already been clinically applied. However, the mite allergen-specific sublingual immunotherapy has not been found to have efficacy for asthma patients. There is a need to develop an additive (adjuvant or allergenic action enhancer) for enhancing an action of the current sublingual immunotherapy with a mite allergen. In addition, localized itching and swelling in the mouth are found as main side effects of the sublingual immunotherapy, and hence there is a need for amelioration, such as a reduction in amount of the allergen to be administered.

Involvement of regulatory T cells (Tregs) has been suggested as one action mechanism of the sublingual immunotherapy. There is a report that: the regulatory T cells (Tregs) highly express an adhesion molecule CD44 on their cell surfaces and amplify TGF-β signaling via CD44, leading to activation; and a function of the regulatory T cells (Tregs) is lowered in CD44-deficient mice (Non Patent Literature 1). The inventor of the present invention et al. have, for the first time in the world, developed a sublingual immunotherapy mouse model using galectin-9 (Gal-9), a ligand for CD44, and demonstrated efficacy of Gal-9 as an allergenic action enhancer (Non Patent Literature 2). As other ligands for CD44, there are known hyaluronic acid, collagen, fibronectin, laminin, serglycin, and the like (Patent Literature 1 and Patent Literature 2).

Gebe et al. disclose that allergic airway inflammation in mice sensitized to ovalbumin or cockroach proteins can be prevented with XHA (thiolated high molecular weight hyaluronic acid tethered to an allergen via a thiol linkage) (Non Patent Literature 3). This mode, in which an allergen is bound to hyaluronic acid via a thiol linkage, is different from a mode in which hyaluronic acid is used as an allergenic action enhancer in combined use with an allergen.

In a Chinese patent application (Patent Literature 3), there is a disclosure of an allergen-containing film formulation. There is a description that hyaluronic acid is used as a film-forming material in the film formulation, and there is a description that the film-forming material accounts for from 30% to 99% of the entire medicinal composition in terms of mass ratio. However, hyaluronic acid is used as a film-forming material, and there is no disclosure or suggestion that hyaluronic acid may be used as an allergenic action enhancer. In addition, it is known that, in the case where a film is formed with hyaluronic acid, when an average molecular weight of the hyaluronic acid is low, a predetermined viscosity is not achieved by increasing its concentration, and hence the average molecular weight of the hyaluronic acid to be used for an aqueous solution of hyaluronic acid is set to 1×10⁵ or more (see paragraph 0016 of JP 2014-114355 A, hyaluronic acids having average molecular weights of from 100,000 to 800,000 are used in Examples).

### Citation List

### Patent Literature

[PTL 1] JP 2013-144700 A
[PTL 2] JP 2013-195077 A
[PTL 3] CN 102048712

### Non Patent Literature

[NPL 1] Wu C et al., Immunity. 2014 Aug 21; 41(2): 270-82
[NPL 2] Ikeda Metal., Allergol Int. 2017 Jul; 66(3): 432-439
[NPL 3] Gebe JA et al., Am J Respir Cell Mol Biol. 2017 Jan; 56(1): 109-120

### SUMMARY OF INVENTION

### Technical Problem

An object of the present invention is to enhance the action of an allergen in sublingual immunotherapy for an asthma patient, and to reduce the amount of the allergen in order to reduce side effects of the allergen in the sublingual immunotherapy.

### Solution to Problem

The inventor of the present invention has generated, with a view to clinical application to humans, a sublingual immunotherapy mouse model using hyaluronic acid (HA) as a CD44 ligand that can be administered to a human body, and has found the efficacy of hyaluronic acid as an allergenic action enhancer. Thus, the present invention has been completed.

That is, the present invention includes the following:
1. An allergenic action enhancer to be administered in combined use with an allergen and to be used for enhancing an action of the allergen in sublingual immunotherapy (SLIT), the allergenic action enhancer including hyaluronic acid (HA) as an active ingredient.
2. The allergenic action enhancer according to the above-mentioned item 1, wherein the hyaluronic acid (HA) is low molecular weight hyaluronic acid (HA-L) or medium molecular weight hyaluronic acid (HA-M).
3. The allergenic action enhancer according to the above-mentioned item 1 or 2, wherein the allergen is a mite allergen, a cedar allergen, or a ragweed allergen.
4. The allergenic action enhancer according to any one of the above-mentioned items 1 to 3, wherein the sublingual immunotherapy (SLIT) is performed for a patient with an allergic disease.
5. The allergenic action enhancer according to the above-mentioned item 4, wherein the allergic disease is allergic rhinitis or asthma.
6. The allergenic action enhancer according to any one of the above-mentioned items 1 to 5, wherein the allergenic action enhancer is an allergenic action enhancer for suppressing production of IL-5, IL-13, and/or an IgE antibody.
7. The allergenic action enhancer according to any one of the above-mentioned items 1 to 6, wherein the allergenic action enhancer is an allergenic action enhancer for promoting production of IL-10 and/or induction of TGF-β-positive regulatory T cells (Tregs).
8. A pharmaceutical composition for oromucosal administration, including an allergen and hyaluronic acid.
9. The pharmaceutical composition according to the above-mentioned item 8, wherein the hyaluronic acid is low molecular weight hyaluronic acid (HA-L) or medium molecular weight hyaluronic acid (HA-M).
10. The pharmaceutical composition according to the above-mentioned item 8 or 9, wherein the allergen is a mite allergen, a cedar allergen, or a ragweed allergen.
11. The pharmaceutical composition according to any one of the above-mentioned items 8 to 10, wherein the pharmaceutical composition is for use in treatment of an allergic disease.
12. The pharmaceutical composition according to the above-mentioned item 11, wherein the allergic disease is allergic rhinitis or asthma.

### Advantageous Effects of Invention

According to the present invention, through combined use or blending of hyaluronic acid as the allergenic action enhancer with the allergen, the effect of allergen-specific sublingual immunotherapy can be enhanced, leading to the cure of asthma, in particular, atopic asthma.

In addition, according to the present invention, through enhancement of the allergenic action, the sublingual immunotherapy (SLIT) can be performed with a smaller amount of the allergen.

### Brief Description of Drawings

FIG. 1 shows suppressing actions on eosinophilic airway inflammation found by using mite allergen-induced chronic asthma model mice and performing sublingual immunotherapy through addition of hyaluronic acid (HA) (Example 6).
FIG. 2 shows suppressing actions on localized IL-5 production in the airway found by using mite allergen-induced chronic asthma model mice and performing sublingual immunotherapy through addition of hyaluronic acid (HA) (Example 6).
FIG. 3 shows suppressing actions on localized IL-13 production in the airway found by using mite allergen-induced chronic asthma model mice and performing sublingual immunotherapy through addition of hyaluronic acid (HA) (Example 6).
FIG. 4 shows lowering actions on a serum mite-specific IgE antibody titer found by using mite allergen-induced chronic asthma model mice and performing sublingual immunotherapy through addition of hyaluronic acid (HA) (Example 6).
FIG. 5 shows the results of sublingual immunotherapy performed using hyaluronic acid (HA) as an allergenic action enhancer in mite allergen-induced chronic asthma model mice (Example 6).
FIG. 6 shows CD44-dependent production of antigen-specific IL-10 in antigen-sensitized spleen cells in vitro (Example 6).
FIG. 7 shows CD44-dependent induction of TGF-β-positive Tregs in antigen-sensitized spleen cells in vitro (Example 6).
FIG. 8 shows suppressing actions on eosinophilic airway inflammation found by using mite allergen-induced chronic asthma model mice and performing sublingual immunotherapy through addition of hyaluronic acids (HA) having different molecular weights (Example 7).
FIG. 9 shows the results of an investigation on a mite-specific IgE antibody titer in blood by performing sublingual immunotherapy using hyaluronic acids (HA) having different molecular weights as allergenic action enhancers in mite allergen-induced chronic asthma model mice (Example 7).
FIG. 10 shows the results of an airway hyperresponsiveness examination performed after sublingual immunotherapy using hyaluronic acids (HA) having different molecular weights as allergenic action enhancers in mite allergen-induced chronic asthma model mice (Example 7).
FIG. 11 shows the results of sublingual immunotherapy performed using medium molecular weight hyaluronic acid (HA-M) as an allergenic action enhancer in mite allergen-induced chronic asthma model mice (Example 7).
FIG. 12 shows results obtained by culturing spleen cells of mice in which cedar sensitization has been established with a cedar allergen (Ced), in the presence of the cedar allergen or the cedar allergen and medium molecular weight hyaluronic acid (HA-M), and measuring IL-10 in the culture supernatant by an ELISA method (Example 8).
FIG. 13 shows results obtained by culturing spleen cells of mice in which ragweed sensitization has been established with a ragweed allergen (RW), in the presence of the ragweed allergen or the ragweed allergen and medium molecular weight hyaluronic acid (HA-M), and measuring IL-10 in the culture supernatant by an ELISA method (Example 9).

### Description of Embodiments

Sublingual immunotherapy (SLIT) has the potential to become a more inexpensive and more simple treatment method, and is highly safe with little chance of a systemic response. Besides, its effect has been recognized through a plurality of large-scale investigations in recent years, and its basic theoretical model has been validated. The general effect of the sublingual immunotherapy (SLIT) is based on the observation that the oral mucosa is particularly primed in mammals for development of allergic tolerance and contains specialized cells which function to achieve this. Antigen-presenting cells (APCs) are the initiators of the immune response and are present in the surface of tissues in communication with the environment. The antigen-presenting cells (APCs) capture allergens and present the allergens to T cells. At this time, it is presumed that the presence of hyaluronic acid promotes the induction of regulatory T cells via CD44, suppresses cytokine production from Th2 cells and IgE production from B cells, and induces tolerance to the allergens.

Herein, the term "allergen (antigen)" refers to any substance that triggers the production of IgE measurable in at least part of a patient exposed to the allergen. The allergen is preferably an allergen to be used for immune tolerance in the oral mucosa, and more preferred examples thereof include a mite allergen, an allergen for cedar hay fever (herein sometimes referred to as "cedar allergen" or "Ced"), and an allergen for ragweed hay fever (herein sometimes referred to as "ragweed allergen" or "RW"). An allergen for pollen may be used for the treatment of its hay fever. A suitable example of the mite allergen may be "MITICURE (trademark) House Dust Mite Sublingual Tablets" available on the market from Torii Pharmaceutical Co., Ltd. In addition, a suitable example of the allergen for cedar hay fever may be "CEDARTOLEN (trademark) SUBLINGUAL DROP - Japanese Cedar Pollen" available on the market from Torii Pharmaceutical Co., Ltd. A wide range of other known allergens available on the market may also be applied.

A production method for an allergen extract, a purification method for allergen molecules, and the like are well known in the art. A brief description is now given. A raw material for an allergen (e.g., pollen, an insect, or scales) may be first pulverized, dried, and defatted (through extraction with an organic solvent), or subjected to any other process appropriate for each allergen. For example, centrifugation may be used in order to separate a solid or particulate material. The resultant material may be incubated in an aqueous medium (e.g., water or an appropriate buffer, such as ammonium bicarbonate buffer or phosphate buffered saline) for a period of time appropriate for at least partially solubilizing a protein. A crude extract may be treated by, for example, dialysis, filtration, fractionation, or chromatography. In some embodiments, for example, one or more processes are performed in order to remove at least part of low molecular weight components or to concentrate the extract. The extract may be sterilized by, for example, filtration and/or irradiation. As known in the art, other treatment processes may be performed. A large number of specific protocols may be used.

As used herein, the term "allergenic action enhancer" refers to a pharmaceutical agent for enhancing the action of an allergen in sublingual immunotherapy (SLIT). The allergenic action enhancer is separate and independent from the allergen, is an allergenic action enhancer for enhancing the action of immune tolerance in the oral mucosa by the allergen, and is preferably an allergenic action enhancer for suppressing the production of IL-5, IL-13, or an IgE antibody. It is known that, in an allergic disease, an allergic reaction occurs through an increase in IgE antibody in the body. In addition, type 2 helper T cells (TH2) are known to produce cytokines IL-5 and IL-13, which are involved in allergic inflammation.

Herein, the term "regulatory T cells (Tregs)" refers to CD4+ T cells identified as immunosuppressive subsets and expressing a transcription factor Foxp3. The regulatory T cells (Tregs) are known to play an important role in maintaining immunological homeostasis. Further, the regulatory T cells (Tregs) are known to highly express an immunosuppressive cytokine IL-10.

In addition, TGF-β is known to play an important role in regulating the development of the regulatory T cells (Tregs).

Herein, the term "combined use" refers to the use of the allergenic action enhancer according to the present invention, which is separate and independent from the allergen, in a subject before or after the use of the allergen or simultaneous with the use of the allergen, in which the allergenic action enhancer and the allergen only need to be brought into a state of coexisting in the same action region. Herein, the term "blend" means formulating by mixing at a certain ratio in a so-called pharmaceutical formulation.

As used herein, the term "hyaluronic acid (HA)" refers to hyaluronic acid having a function of enhancing the action of an allergen in sublingual immunotherapy (SLIT), and encompasses high molecular weight hyaluronic acid (HA-H), medium molecular weight hyaluronic acid (HA-M), and low molecular weight hyaluronic acid (HA-L). The hyaluronic acid (HA) may be a complex containing hyaluronic acid, a salt of hyaluronic acid, or a hyaluronic acid derivative (excluding a mode of being bound to the allergen).

As used herein, the term "high molecular weight hyaluronic acid (HA-H)" refers to hyaluronic acid having a weight average molecular weight of from 500,000 to 1,000,000, the term "medium molecular weight hyaluronic acid (HA-M)" refers to hyaluronic acid having a weight average molecular weight of from 40,000 to 65,000, and the term "low molecular weight hyaluronic acid (HA-L)" refers to hyaluronic acid having a weight average molecular weight of from 3,600 to 4,400. Herein, the molecular weight means an absolute molecular weight based on a GPC-MALS method.

As used herein, the term "allergic disease" refers to a disease developed by allergen stimulation, and examples thereof include allergic rhinitis, asthma, hay fever, atopic dermatitis, and a food allergy. Of those, allergic rhinitis or asthma is preferred, and atopic asthma is more preferred.

As used herein, the term "treatment" refers to a medical intervention for ameliorating symptoms of an allergic disease, and examples thereof include immunotherapy and pharmacotherapy. Of those, sublingual immunotherapy is preferred.

One embodiment of the present invention relates to an allergenic action enhancer (hereinafter sometimes referred to as "agent") to be administered in combined use with an allergen and to be used for enhancing an action of the allergen in sublingual immunotherapy (SLIT), the allergenic action enhancer containing hyaluronic acid (HA) as an active ingredient.

Another embodiment of the present invention relates to a pharmaceutical composition for oromucosal administration (hereinafter sometimes referred to as "pharmaceutical composition"), containing an allergen and hyaluronic acid having an allergenic action-enhancing ability.

The agent or the pharmaceutical composition of the present invention is oromucosally administered, preferably sublingually administered. A formulation for oromucosal administration generally has the dosage form of a solution obtained by dissolving an active amount of an active ingredient in a diluent, such as water or saline, a suspension, an emulsion, a troche, or a tablet that can be sublingually held for several minutes and then swallowed. In order to promote absorption into the body through the oral mucosa, an osmotic pressure ratio may be adjusted to a value of more than 1 (for example, from 1.1 to 10.0, preferably from 1.1 to 7, more preferably from 1.5 to 5, still more preferably from 2 to 4 (e.g., 3)). Osmotic pressure adjustment or stabilization may be performed by adding an isotonic agent, such as glycerin, glucose, or sodium chloride, into the formulation. When an accurate osmotic pressure ratio cannot be measured owing to the presence of an additive, the value of an osmotic pressure ratio at 10-fold dilution with distilled water is adjusted to fall within the above-mentioned numerical ranges.

With regard to the allergen for combined use in the agent of the present invention, the content of the allergen in the formulation or the pharmaceutical composition varies depending on, for example, the kind of the allergen and the form of the formulation, but generally falls within the range of from 0.1 ng/ml to 1 mg/ml, preferably from 1 ng/ml to 0.1 mg/ml. In Japan, the content of an allergen in an allergen composition is expressed in Japanese allergy units (JAU) set by the Japanese Society of Allergology on the basis of skin testing of allergic patients. Specifically, in the case of a mite allergen, a total concentration of Der f 1 (main allergen of *Dermatophagoides farinae)* and Der p 1 (main allergen of *Dermatophagoides pteronyssinus)* within the range of from 22.2 µg/ml to 66.7 µg/ml may be expressed as 100,000 JAU/mL, and in the case of a cedar pollen allergen, a concentration of Cryjl within the range of from 7.3 µg/ml to 21 µg/ml may be expressed as 10,000 JAU/mL.

The content ratio of the hyaluronic acid serving as an active ingredient in the entirety of the agent or the pharmaceutical composition of the present invention is from 0.001% to 5%, preferably from 0.02% to 1%, more preferably from 0.04% to 0.5% of the weight of the formulation.

The weight ratio which the hyaluronic acid serving as an active ingredient is used at in the agent of the present invention or accounts for in the entirety of the pharmaceutical composition with respect to the allergen is from 0.1 to 5,000, preferably from 50 to 2,000 with respect to 100 of the weight of the allergen, and may be set to from 80 to 600.

The agent or the pharmaceutical composition of the present invention may be manufactured by: mixing the hyaluronic acid serving as an active ingredient with one or more kinds of pharmacologically acceptable carriers; and employing any method well known in the technical field of pharmaceutics.

As the pharmacologically acceptable carriers, there are used various organic or inorganic carrier substances commonly used as formulation materials. Specific examples thereof include: an excipient, a lubricant, a binder, and a disintegrant in a solid formulation; and a solvent, a solubilizing agent, a suspending agent, a tonicity agent, a buffer, and a soothing agent in a liquid formulation. In formulating, as required, there may be used a formulation additive, such as an antiseptic agent, an antioxidant, a colorant, or a sweetening agent.

Examples of the pharmacologically acceptable additive include, but not limited to: excipients, such as sucrose, starch, mannitol, sorbitol, lactose, glucose, cellulose, talc, calcium phosphate, and calcium carbonate; binders, such as cellulose, methyl cellulose, hydroxypropyl cellulose, polypropylpyrrolidone, gelatin, gum arabic, polyethylene glycol, sucrose, and starch; disintegrants, such as starch, carmellose, hydroxypropyl starch, sodium-glycol-starch, sodium hydrogen carbonate, calcium phosphate, and calcium citrate; lubricants, such as magnesium stearate, talc, and sodium lauryl sulfate; flavoring agents, such as citric acid, menthol, diammonium glycyrrhizinate, glycine, and orange oil; preservatives, such as sodium benzoate, sodium hydrogen sulfite, methylparaben, and propylparaben; stabilizing agents, such as citric acid, sodium citrate, and acetic acid; suspending agents, such as methyl cellulose, povidone, and aluminum stearate; dispersants, such as a surfactant; diluents, such as water, physiological saline, and orange juice; and base waxes, such as cacao butter, polyethylene glycol, and white kerosene.

When the agent or the pharmaceutical composition of the present invention is oromucosally administered, preferably sublingually administered, a mode of an administration schedule is as described below. An administration frequency is, for example, once every 1 to 7 days, preferably once every 1 to 3 days, most preferably once a day. An administration period may be appropriately set while checking a therapeutic effect, but is generally 3 months or more, preferably 5 months or more. The upper limit of the administration period may also be appropriately set while checking the therapeutic effect, but the administration is generally continued until at least the end of the dispersal of pollen responsible for hay fever is recognized. The administration may be continued thereafter until a satisfactory therapeutic effect is recognized. The upper limit of the administration period is also not particularly limited, and long-term administration for 2 years or more is also recommended.

With regard to the dose of the agent or the pharmaceutical composition of the present invention, in order to avoid risks of exacerbating allergic symptoms and inducing anaphylaxis due to allergen administration, the administration may be started at a low dose, followed by an incremental increase in dose. For example, at the start of the administration, the responsiveness (threshold) of a patient positive for a skin reaction is determined through an intradermal reaction. The initial dose is set depending on the threshold and the symptoms of the patient at the time. For example, a dilution series of the allergen is prepared, a certain amount thereof is intradermally administered to a subject, a reaction thereof is judged in accordance with intradermal reaction criteria. The lowest concentration (maximum rate of dilution) at which a positive reaction has been exhibited is adopted as the threshold of the subject for the allergen. The initial dose is set so as to be lower (e.g., from 1/10 to 1/100) than the dose at the threshold, and the dose is incrementally increased. Buccal (sublingual) administration has relatively high safety, and hence it is preferred that: the administration be started at a predetermined certain amount, followed by an incremental increase in dose; or the same dose be kept over the period from the beginning to the end of the administration.

The subject to which the agent or the pharmaceutical composition of the present invention is to be administered is generally a mammal. Examples of the mammal may include, but not limited to: experimental animals including rodents, such as a mouse, a rat, a hamster, and a guinea pig, and a rabbit; livestock, such as a pig, a bovine, a goat, a horse, a sheep, and a mink; pet animals, such as a dog and a cat; and primates, such as a human, a monkey, a crab-eating macaque, a rhesus macaque, a marmoset, an orangutan, and a chimpanzee. The mammal is preferably a primate (e.g., a human) or a rodent (e.g., a mouse).

In one embodiment of the present invention, the allergenic action enhancer containing hyaluronic acid (HA) as an active ingredient is a pharmaceutical agent for enhancing the action of an allergen in sublingual immunotherapy (SLIT), only needs to be an allergenic action enhancer for enhancing the action of immune tolerance in the oral mucosa by the allergen, is separate and individual from the allergen, and is administered in combined use or as a blend with the allergen.

In one embodiment of the present invention, the hyaluronic acid (HA) contained as an active ingredient in the allergenic action enhancer only needs to have a function of enhancing the action of an allergen in sublingual immunotherapy (SLIT), may include high molecular weight hyaluronic acid (HA-H), medium molecular weight hyaluronic acid (HA-M), and low molecular weight hyaluronic acid (HA-L), and may be a complex containing hyaluronic acid, a salt of hyaluronic acid, or a hyaluronic acid derivative (excluding a mode of being bound to the allergen). The hyaluronic acid has preferably an average molecular weight of from 1,600 to 1,000,000, more preferably an average molecular weight of from 3,000 to 500,000, still more preferably an average molecular weight of from 3,600 to 4,400 and/or an average molecular weight of 40,000 to 65,000.

In one embodiment of the present invention, the allergen to be administered in combined use or as a blend with the allergenic action enhancer only needs to trigger the production of IgE measurable in at least part of a patient exposed to the allergen, is an allergen to be used for immune tolerance in the oral mucosa, and may be a mite allergen, a cedar allergen, or a ragweed allergen.

In one embodiment of the present invention, in the allergenic action enhancer containing hyaluronic acid (HA) as an active ingredient, the sublingual immunotherapy (SLIT) is performed for a patient with an allergic disease, and the allergic disease refers to a disease developed by allergen stimulation, may be any one disease selected from the group consisting of allergic rhinitis, asthma, hay fever, atopic dermatitis, and a food allergy, and is preferably allergic rhinitis or asthma, more preferably atopic asthma.

In one embodiment of the present invention, the allergenic action enhancer containing hyaluronic acid (HA) as an active ingredient may be an allergenic action enhancer for suppressing the production of a cytokine or an antibody involved in allergic inflammation, and may be preferably an allergenic action enhancer for suppressing the production of IL-5, IL-13, or an IgE antibody.

In one embodiment of the present invention, the allergenic action enhancer containing hyaluronic acid (HA) as an active ingredient may exhibit its effect by acting on regulatory T cells (Tregs), or may promote the production of IL-10 and/or the induction of TGF-β-positive regulatory T cells (Tregs).

In one embodiment of the present invention, the pharmaceutical composition for oromucosal administration containing hyaluronic acid (HA) as an active ingredient may be preferably a pharmaceutical composition for sublingual administration, which has an ability to enhance the action of an allergen in sublingual immunotherapy (SLIT).

In one embodiment of the present invention, the hyaluronic acid (HA) contained as an active ingredient of the allergenic action enhancer contained in the pharmaceutical composition for oromucosal administration, preferably the pharmaceutical composition for sublingual administration, only needs to have a function of enhancing the action of an allergen in sublingual immunotherapy (SLIT), may include high molecular weight hyaluronic acid (HA-H), medium molecular weight hyaluronic acid (HA-M), and low molecular weight hyaluronic acid (HA-L), and may be a complex containing hyaluronic acid, a salt of hyaluronic acid, or a hyaluronic acid derivative (excluding a mode of being bound to the allergen). The hyaluronic acid has preferably an average molecular weight of from 1,600 to 1,000,000, more preferably an average molecular weight of from 3,000 to 500,000, still more preferably an average molecular weight of from 3,600 to 4,400 and/or an average molecular weight of 40,000 to 65,000.

In one embodiment of the present invention, the allergen contained in the pharmaceutical composition for oromucosal administration, preferably the pharmaceutical composition for sublingual administration, only needs to trigger the production of IgE measurable in at least part of a patient exposed to the allergen, may be an allergen to be used for immune tolerance in the oral mucosa, and may be a mite allergen, a cedar allergen, or a ragweed allergen.

In one embodiment of the present invention, the pharmaceutical composition for oromucosal administration, preferably the pharmaceutical composition for sublingual administration, may be for use in treatment of a patient with an allergic disease. The allergic disease may be a disease developed by allergen stimulation, may be any one disease selected from the group consisting of allergic rhinitis, asthma, hay fever, atopic dermatitis, and a food allergy, is preferably allergic rhinitis or asthma, and may be more preferably atopic asthma.

In one embodiment of the present invention, the pharmaceutical composition for oromucosal administration, preferably the pharmaceutical composition for sublingual administration, may be a pharmaceutical composition for suppressing the production of a cytokine or an antibody involved in allergic inflammation, and may be preferably a pharmaceutical composition for suppressing the production of IL-5, IL-13, or an IgE antibody.

In one embodiment of the present invention, the pharmaceutical composition for oromucosal administration, preferably the pharmaceutical composition for sublingual administration, may exhibit its effect by acting on regulatory T cells (Tregs), or may, for example, promote the production of IL-10 and/or the induction of TGF-β-positive regulatory T cells (Tregs).

One embodiment of the present invention relates to an allergenic action enhancement method, including administering hyaluronic acid (HA) together with an allergen, and the administration is preferably sublingual administration. The "allergenic action enhancement" refers to enhancing the action of the allergen in sublingual immunotherapy (SLIT). The hyaluronic acid (HA) only needs to have a function of enhancing the action of the allergen in the sublingual immunotherapy (SLIT). Such hyaluronic acid (HA) encompasses high molecular weight hyaluronic acid (HA-H), medium molecular weight hyaluronic acid (HA-M), and low molecular weight hyaluronic acid (HA-L), preferably medium molecular weight hyaluronic acid (HA-M) and low molecular weight hyaluronic acid (HA-L).

One embodiment of the present invention relates to a method of treating an allergic disease, including sublingually administering hyaluronic acid (HA) together with an allergen. The hyaluronic acid (HA) only needs to have a function of enhancing the action of the allergen in sublingual immunotherapy (SLIT). The allergic disease is preferably allergic rhinitis or asthma, more preferably atopic asthma.

One embodiment of the present invention relates to hyaluronic acid (HA) for enhancing an action of an allergen in sublingual immunotherapy (SLIT). The hyaluronic acid (HA) only needs to have a function of enhancing the action of the allergen in the sublingual immunotherapy (SLIT). Such hyaluronic acid (HA) encompasses high molecular weight hyaluronic acid (HA-H), medium molecular weight hyaluronic acid (HA-M), and low molecular weight hyaluronic acid (HA-L), preferably medium molecular weight hyaluronic acid (HA-M) and low molecular weight hyaluronic acid (HA-L).

One embodiment of the present invention relates to hyaluronic acid (HA) for treating an allergic disease. The hyaluronic acid (HA) is preferably used together with an allergen, and it is more preferred that the hyaluronic acid (HA) be sublingually administered together with an allergen. The hyaluronic acid (HA) only needs to have a function of enhancing the action of the allergen in sublingual immunotherapy (SLIT). The allergic disease is preferably allergic rhinitis or asthma, more preferably atopic asthma.

The present invention is described in detail below by way of Examples, but the technical scope of the present invention is not to be construed as limited by the following description of Examples. A person skilled in the art could make many variations and modifications without departing from the technical scope of the present invention on the basis of the description given herein. In addition, unless otherwise specified, the following Examples may be carried out in accordance with standard techniques in genetic engineering and molecular biology known to a person skilled in the art as described in, for example, Sambrook and Maniatis, in Molecular Cloning-A Laboratory Manual, Cold Spring Harbor Laboratory Press, New York, 1989; Ausubel, F. M. et al., Current Protocols in Molecular Biology, John Wiley & Sons, New York, N.Y, 1995. In addition, when a kit is used, the kit's instructions are followed. The contents described in documents cited herein constitute part of the disclosure and contents herein.

### Example 1

### Tablets and Granule Formulations and Sublingual Formulations

4 mg of hyaluronic acid having an average molecular weight of 4,000, 100 mg of citric acid, 300 mg of sucrose, and 10 mg of PEG 4,000 were placed in a mortar, 5 ml of purified water was added, and the contents were mixed well with a pestle. While the contents were stirred under ordinary temperature and reduced pressure, water was evaporated, and then the residue was formed into minitablets (tablets). In addition, the contents in a state of retaining viscosity were extruded through use of a mesh to prepare granules.

### Example 2

### Tablets

4 mg of hyaluronic acid having an average molecular weight of 30,000, 50 mg of citric acid, and 150 mg of low-substituted hydroxypropyl cellulose are thoroughly mixed in a mortar with a pestle, and then formed into ten tablets by a direct compression method.

### Example 3

### Tablets

4 mg of hyaluronic acid having an average molecular weight of 15,000, 50 mg of citric acid, and 150 mg of EXPLOTAB were thoroughly mixed in a mortar with a pestle, and then formed into ten tablets by a direct compression method.

### Example 4

### Film Formulation

1 mg of hyaluronic acid having an average molecular weight of 500,000, 100 mg of hydroxypropyl cellulose, and 30 mg of polyethylene glycol 300 were dissolved using purified water as a solvent, and then prepared into two mucosa-adhesive films.

### Example 5

### Tablets

Example 2 was repeated, and further, as an allergen, 125 µg or 250 µg of a mite allergen extract was added to prepare tablets.

### Example 6

### Sublingual Immunotherapy (SLIT) through Addition of Hyaluronic Acid (HA) with Use of Mite Allergen-induced Chronic Asthma Model Mice

Mice (BALB/c) were intranasally administered a mite *(Dermatophagoides farinae* (Df)) extract (Torii Pharmaceutical Co., Ltd.) for 3 weeks (5 days/week) to prepare mite allergen-induced chronic asthma model mice. After that, sublingual immunotherapy (SLIT) was performed by sublingually administering 10 µl of the following for 2 weeks (5 days/week): the mite allergen extract (125 µg/day); the mite allergen extract and galectin-9 (Gal-9, 0.3 µg) (GalPharma Co., Ltd.); the mite allergen extract and low molecular weight hyaluronic acid (HA-L, having an average molecular weight of 4,000 or an average molecular weight of about 4,000, 10 µg/day) (HYALOSE); and the mite allergen extract and high molecular weight hyaluronic acid (HA-H, having an average molecular weight of 500,000 or an average molecular weight of about 500,000, 10 µg/day) (HYALOSE). As a negative control, PBS was administered. It was recognized with trypan blue that the allergen used for the sublingual immunotherapy (SLIT) was not delivered to the stomach or the lungs. After 3 days from the final sublingual treatment, the mice were nasally exposed to the mite extract under anesthesia. After 24 hours from the final allergen exposure, an airway hyperresponsiveness examination was performed, and then bronchoalveolar lavage (BAL) was performed. In the airway hyperresponsiveness examination, the rate of increase in airway resistance after methacholine inhalation was measured. The bronchoalveolar lavage (BAL) method was performed in accordance with a conventional method. The number of inflammatory cells and cytokine levels in bronchoalveolar lavage fluid (BALF), and serum IgE were analyzed. Cells in the bronchoalveolar lavage fluid (BALF) were counted using a cell counter or a FACS flow cytometer. IL-5 and IL-13 in the bronchoalveolar lavage fluid (BALF) and serum IgE were measured by ELISA.

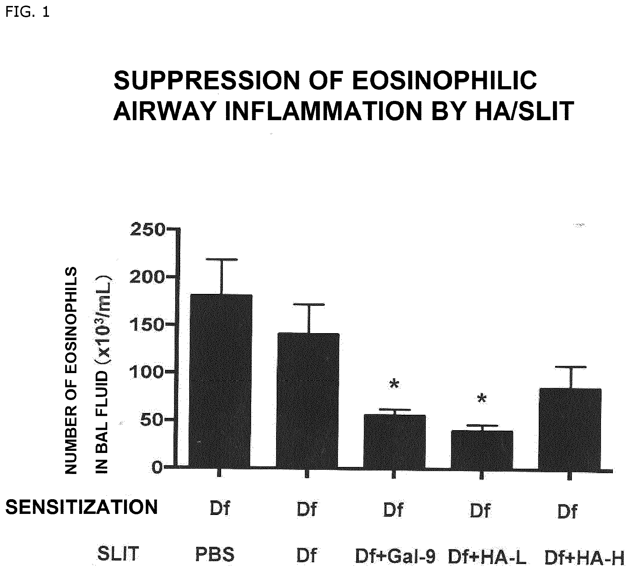

As a result of the sublingual immunotherapy using hyaluronic acid (HA) as an allergenic action enhancer in mite allergen-induced chronic asthma model mice, suppressing effects on eosinophilic airway inflammation were found (FIG. 1, "*" represents P<0.05). In particular, the sublingual immunotherapy (SLIT) with the mite allergen extract and low molecular weight hyaluronic acid (HA-L) had a high suppressing effect on the number of eosinophils.

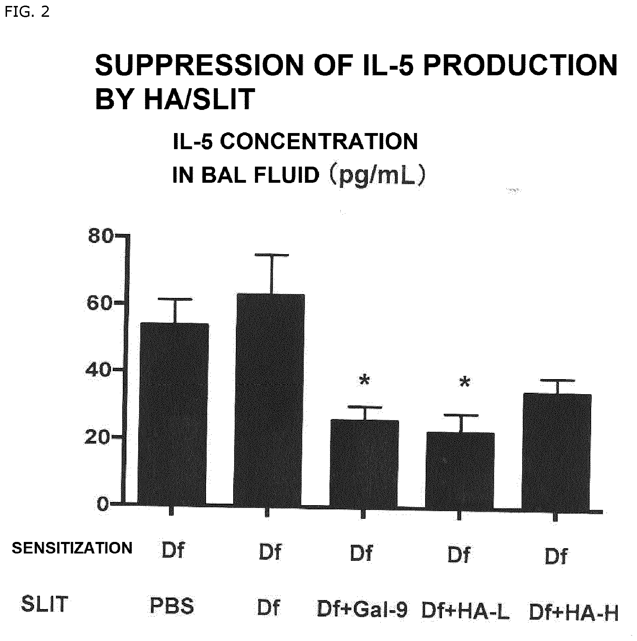

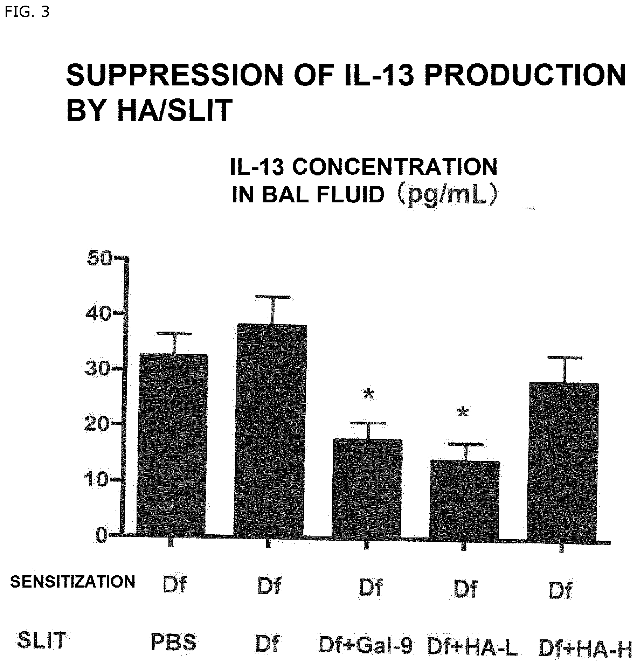

As a result of the sublingual immunotherapy using hyaluronic acid (HA) as an allergenic action enhancer in mite allergen-induced chronic asthma model mice, suppressing effects on the localized production of IL-5 in the airway (FIG. 2, "*" represents P<0.05) and suppressing effects on the localized production of IL-13 in the airway (FIG. 3, "*" represents P<0.05) were found. In particular, suppressing effects were high on the production of IL-5 and IL-13, cytokines involved in allergic inflammation, in the bronchoalveolar lavage fluid (BALF) after the sublingual immunotherapy (SLIT) with the mite allergen extract and low molecular weight hyaluronic acid (HA-L).

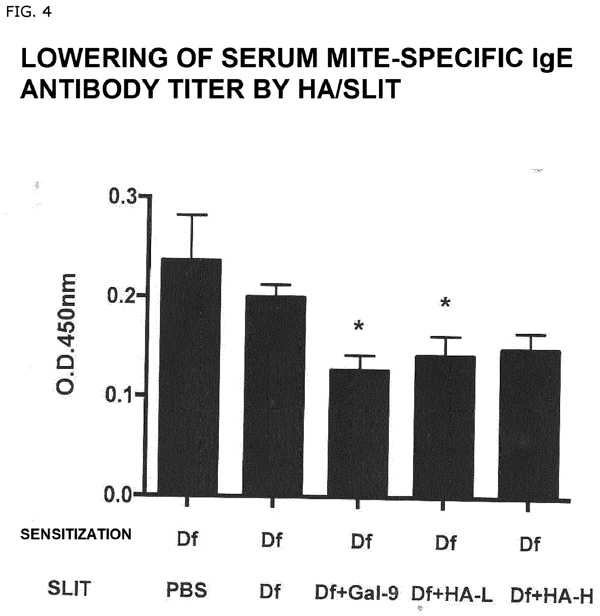

As a result of the sublingual immunotherapy using hyaluronic acid (HA) as an allergenic action enhancer in mite allergen-induced chronic asthma model mice, suppressing effects on a mite-specific IgE antibody titer in blood were found (FIG. 4, "*" represents P<0.05).

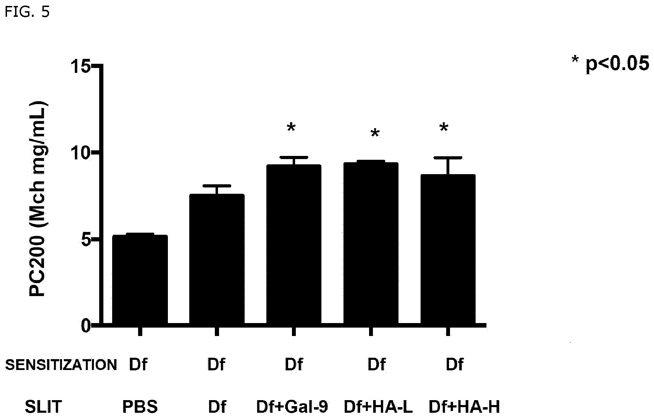

As a result of the sublingual immunotherapy using hyaluronic acid (HA) as an allergenic action enhancer in mite allergen-induced chronic asthma model mice, suppressing effects on airway hyperresponsiveness were found (FIG. 5, "*" represents P<0.05). For the evaluation of airway hyperresponsiveness, airway resistance after methacholine inhalation was measured. A methacholine concentration (PC200) at which the airway resistance is doubled is shown. The methacholine concentration (PC200) was increased by the effect of the sublingual immunotherapy (SLIT), suggesting that the airway hyperresponsiveness was ameliorated.

An action mechanism was investigated as described below. Spleen cells obtained from mice sensitized with the mite allergen were cultured in the presence of the mite allergen. The spleen cells were collected by a conventional method, hemolyzed, and then cultured in a liquid medium (RPMI1640+10%FCS) in a CO₂ incubator (4% CO₂) at 37°C for 24 hours. As a control, a rat IgG2a antibody (RIgG2a) (CEDARLANE) or an anti-CD44 monoclonal antibody (aCD44mAb) (KM81: CEDARLANE) serving to inhibit interaction with CD44 was added to the medium, and the concentration of IL-10 known to be produced by regulatory T cells (Tregs) was measured by ELISA.

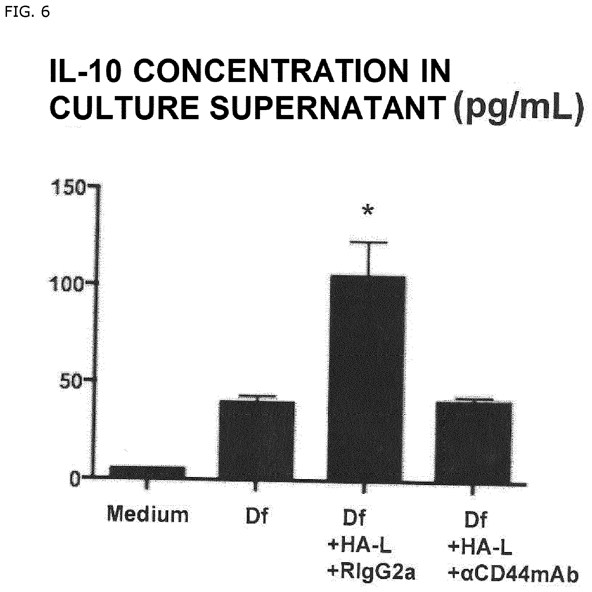

In in-vitro allergen stimulation of the spleen cells obtained from mice sensitized with the mite allergen, in contrast to the rat IgG2a antibody (RIgG2a), the addition of the anti-CD44 monoclonal antibody (aCD44mAb) serving to inhibit interaction with CD44 inhibited the IL-10 production-promoting effect of low molecular weight hyaluronic acid (HA-L). Therefore, the hyaluronic acid (HA) promoted CD44-dependent production of allergen-specific IL-10 by the spleen cells (FIG. 6, "*" represents P<0.05).

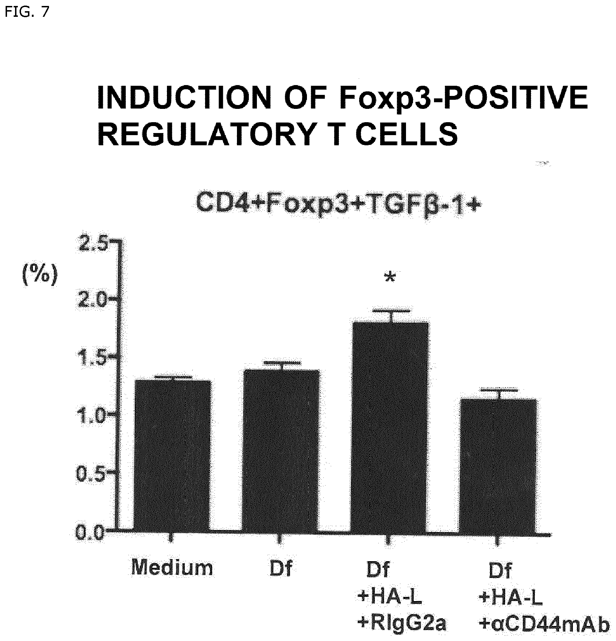

TGF-β-positive CD4-positive Foxp3-positive regulatory T cells (Tregs) were counted with a Treg staining kit making use of a FACS flow cytometer. The spleen cells sensitized with the mite allergen promoted the induction of TGF-β-positive CD4-positive Foxp3-positive regulatory T cells in the presence of low molecular weight hyaluronic acid (HA-L) in vitro (FIG. 7, "*" represents P<0.05). In addition, in contrast to the rat IgG2a antibody (RIgG2a), the addition of the anti-CD44 monoclonal antibody (aCD44mAb) serving to inhibit interaction with CD44 inhibited the promoting effect on the induction of TGF-β-positive CD4-positive Foxp3-positive regulatory T cells (FIG. 7, "*" represents P<0.05). Therefore, it may be said that hyaluronic acid (HA) induces regulatory T cells via CD44 in spleen cells.

### Example 7

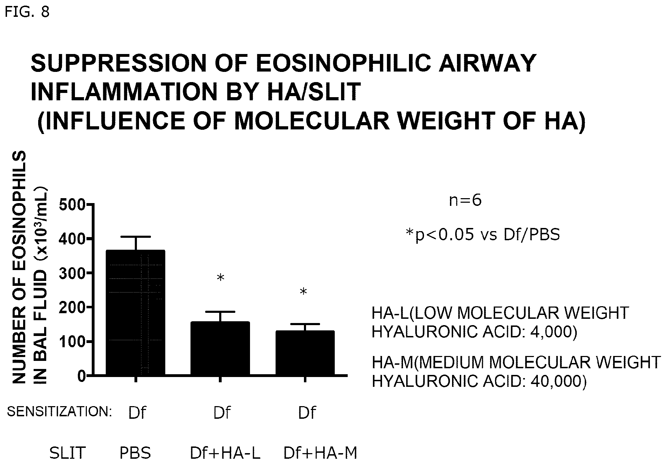

In the same manner as in Example 6, sublingual immunotherapy was performed in mite allergen-induced chronic asthma model mice using a mite *(Dermatophagoides farinae* (Df)) extract (Torii Pharmaceutical Co., Ltd.) and using low molecular weight hyaluronic acid (HA-L, having an average molecular weight of about 4,000, product name: "Hyaluronic acid Oligosaccharide: HA10", Iduro Ltd) and medium molecular weight hyaluronic acid (HA-M, having an average molecular weight of about 44,700 (molecular weights of from 41,000 to 65,000), product name: "Sodium Hyaluronate Research Grade: HA40K", Lifecore Biomedical, LLC) as allergenic action enhancers. As a result, suppressing effects on eosinophilic airway inflammation were found (FIG. 8, "*" represents P<0.05 vs Df/PBS). Low molecular weight hyaluronic acid (HA-L) and medium molecular weight hyaluronic acid (HA-M) showed comparable effects in terms of the suppression of the number of eosinophils by the sublingual immunotherapy (SLIT).

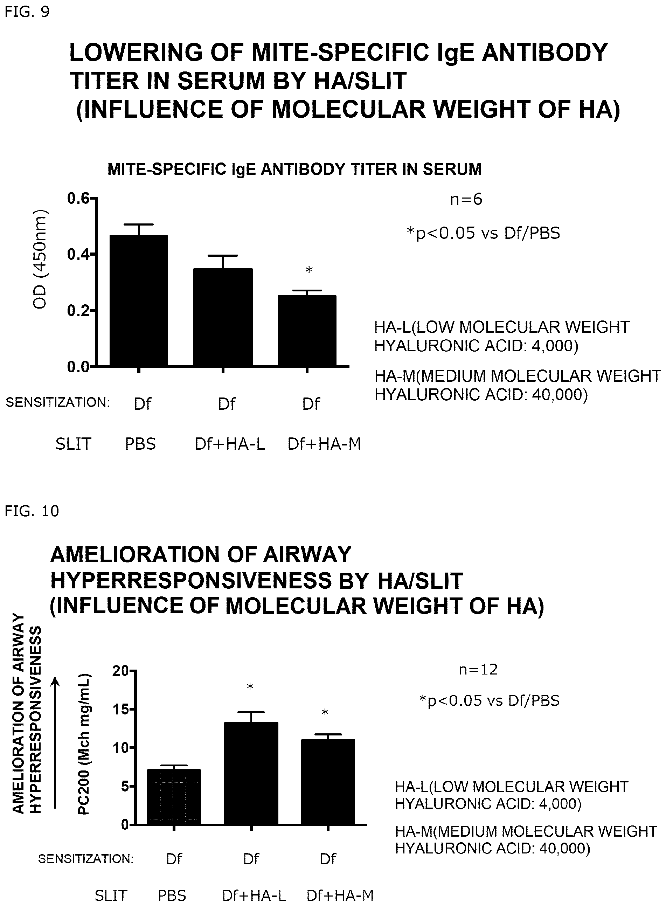

As a result of the sublingual immunotherapy using low molecular weight hyaluronic acid (HA-L, having an average molecular weight of about 4,000) and medium molecular weight hyaluronic acid (HA-M, having an average molecular weight of about 40,000) as allergenic action enhancers in mite allergen-induced chronic asthma model mice, decreasing effects on a mite-specific IgE antibody titer in blood were found (FIG. 9, "*" represents P<0.05 vs Df/PBS). A higher suppressing effect was achieved with medium molecular weight hyaluronic acid (HA-M) than with low molecular weight hyaluronic acid (HA-L).

An airway hyperresponsiveness examination was performed after the sublingual immunotherapy using low molecular weight hyaluronic acid (HA-L, having an average molecular weight of about 4,000) and medium molecular weight hyaluronic acid (HA-M, having an average molecular weight of about 40,000) as allergenic action enhancers in mite allergen-induced chronic asthma model mice. In the airway hyperresponsiveness examination, the rate of increase in airway resistance after methacholine inhalation was measured. As a result, the sublingual immunotherapy (SLIT) with hyaluronic acid (HA) increased the methacholine concentration (PC200), and hence ameliorated airway hyperresponsiveness (FIG. 10, "*" represents P<0.05 vs Df/PBS). Low molecular weight hyaluronic acid (HA-L) and medium molecular weight hyaluronic acid (HA-M) showed comparable effects on airway hyperresponsiveness in the sublingual immunotherapy (SLIT).

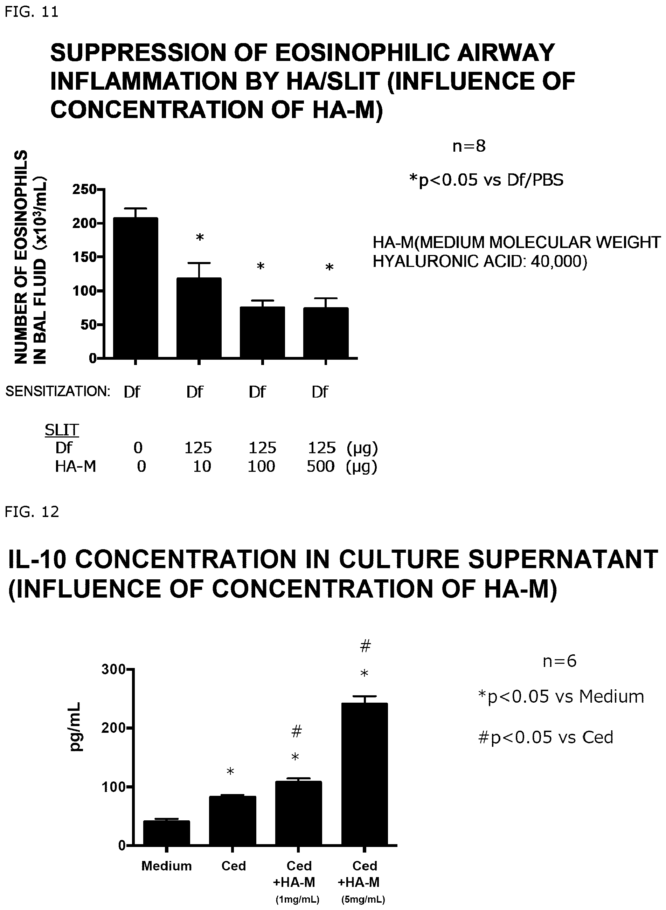

As a result of the sublingual immunotherapy using medium molecular weight hyaluronic acid (HA-M, having an average molecular weight of about 40,000) as an allergenic action enhancer in mite allergen-induced chronic asthma model mice, suppressing effects on eosinophilic airway inflammation were found (FIG. 11, "*" represents P<0.05 vs Df/PBS). Medium molecular weight hyaluronic acid (HA-M) at a high concentration (100 µg or 500 µg) showed a high suppressing effect on the number of eosinophils in the sublingual immunotherapy (SLIT).

### Example 8

A cedar allergen (Ced) (product name: "Cedar Pollen Extract-Ja", LSL) was intraperitoneally administered to mice together with alum once a week for 2 weeks, i.e., twice in total. Spleen cells were collected from the spleen of the mice in which cedar sensitization had been established, and were cultured in a liquid medium (RPMI1640+10%FCS) in the presence of the cedar allergen or the cedar allergen and medium molecular weight hyaluronic acid (HA-M, having an average molecular weight of about 40,000) at 37°C for 48 hours, and IL-10 in the culture supernatant was measured by an ELISA method.

When the sensitized mouse spleen cells were stimulated with the cedar allergen (Ced) in vitro, IL-10 was produced. Medium molecular weight hyaluronic acid (HA-M) promoted Ced-induced IL-10 production in a concentration-dependent manner (FIG. 12, "*" represents P<0.05 vs the medium, and "#" represents P<0.05 vs Ced).

### Example 9

A ragweed allergen (RW) (product name: "Ragweed Pollen Extract", LSL) was intraperitoneally administered to mice together with alum once a week for 2 weeks, i.e., twice in total. Spleen cells were collected from the spleen of the mice in which ragweed sensitization had been established, and were cultured in a liquid medium (RPMI1640+10%FCS) in the presence of the ragweed allergen or the ragweed allergen and medium molecular weight hyaluronic acid (HA-M, having an average molecular weight of about 40,000) at 37°C for 48 hours, and IL-10 in the culture supernatant was measured by an ELISA method.

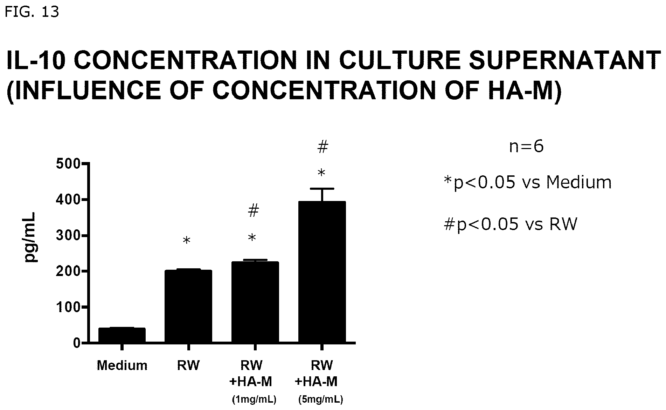

When the sensitized mouse spleen cells were stimulated with the ragweed allergen (RW) in vitro, IL-10 was produced. Medium molecular weight hyaluronic acid (HA-M) promoted RW-induced IL-10 production in a concentration-dependent manner (FIG. 13, "*" represents P<0.05 vs the medium, and "#" represents P<0.05 vs RW).

### Industrial Applicability

The allergenic action enhancer containing hyaluronic acid as an active ingredient and the pharmaceutical composition containing an allergen and hyaluronic acid according to the present invention can be expected to be useful in the treatment of allergic diseases in general by being applied to various allergens in sublingual immunotherapy (SLIT), can enhance an allergenic action in the sublingual immunotherapy (SLIT), leading to the cure of asthma, in particular, atopic asthma, can also be expected to lead to the cure of hay fever through use of cedar pollen or ragweed pollen as the allergen. The allergenic action enhancer containing hyaluronic acid as an active ingredient and the pharmaceutical composition containing an allergen and hyaluronic acid according to the present invention enhance the allergenic action, and hence enable sublingual immunotherapy (SLIT) to be performed with a smaller amount of the allergen. In a paper showing the efficacy of sublingual immunotherapy (SLIT) with a mite allergen using mice as in the present application, the allergen is used in an amount of 0.5 mg or 5 mg (Allergol Int 2017: 66: 89-96). However, in the present invention, the sublingual immunotherapy (SLIT) can be performed with a much smaller amount of the allergen.

## Claims

1. An allergenic action enhancer to be administered in combined use with an allergen and to be used for enhancing an action of the allergen in sublingual immunotherapy (SLIT), the allergenic action enhancer comprising hyaluronic acid (HA) as an active ingredient.

2. The allergenic action enhancer according to claim 1, wherein the hyaluronic acid (HA) is low molecular weight hyaluronic acid (HA-L) or medium molecular weight hyaluronic acid (HA-M).

3. The allergenic action enhancer according to claim 1 or 2, wherein the allergen is a mite allergen, a cedar allergen, or a ragweed allergen.

4. The allergenic action enhancer according to any one of claims 1 to 3, wherein the sublingual immunotherapy (SLIT) is performed for a patient with an allergic disease.

5. The allergenic action enhancer according to claim 4, wherein the allergic disease is allergic rhinitis or asthma.

6. The allergenic action enhancer according to any one of claims 1 to 5, wherein the allergenic action enhancer is an allergenic action enhancer for suppressing production of IL-5, IL-13, and/or an IgE antibody.

7. The allergenic action enhancer according to any one of claims 1 to 6, wherein the allergenic action enhancer is an allergenic action enhancer for promoting production of IL-10 and/or induction of TGF-β-positive regulatory T cells (Tregs).

8. A pharmaceutical composition for oromucosal administration, comprising an allergen and hyaluronic acid.

9. The pharmaceutical composition according to claim 8, wherein the hyaluronic acid is low molecular weight hyaluronic acid (HA-L) or medium molecular weight hyaluronic acid (HA-M).

10. The pharmaceutical composition according to claim 8 or 9, wherein the allergen is a mite allergen, a cedar allergen, or a ragweed allergen.

11. The pharmaceutical composition according to any one of claims 8 to 10, wherein the pharmaceutical composition is for use in treatment of an allergic disease.

12. The pharmaceutical composition according to claim 11, wherein the allergic disease is allergic rhinitis or asthma.
